# EUROPEAN PATENT SPECIFICATION

(11) **EP 1 611 876 B1**
(45) Date of publication and mention of the grant of the patent: **29.02.2012**
(21) Application number: 05013945.0
(22) Date of filing: 28.06.2005
(51) Int. Cl.: A61L 27/48

(54) **Gingival retraction material**
Mittel zur Zahnfleischretraktion
Matériau de rétraction gingivale

(30) Priority: 30.06.2004 CN 200410062893
(43) Date of publication of application: 04.01.2006
(73) Proprietor: Biotech One Inc., Taipei (TW)
(72) Inventor: Yang, Jen-Chang, Taipei (TW); Lee, Hsien-Kun, Taipei (TW); Lee, Sheng-Yang, Taipei (TW); Weng, Ching-Wu, Taipei (TW); Chen, Jiunn-Liang, Taipei (TW); Lin, Hsu-Ting, Taipei (TW)
(74) Representative: Becker Kurig Straus

(56) References cited:
- US-A- 5 362 495
- US-A1- 2003 194 380

## Description

### BACKGROUND OF THE INVENTION

### 1. Field of the Invention

The present invention relates generally to gingival retraction materials for dental treatment and more particularly, to an injectable fibrillated fibers-contained gingival retraction material for widening the gingival sulcus prior to the impression taking.

### 2. Description of the Related Art

In dental therapeutics, gingival retraction procedure is commonly employed to temporarily widen the gingival sulcus for further impressions or for effecting various treatments. The presently known techniques for retracting gingival may be substantially divided into four groups, i.e. the mechanical method, mechanochemical method, rotary curettage method, and the electro-surgery method, depending on the required treatments to the patient's pathology.

Gingival retraction cords, which are inserted into gingival sulcus by a mechanical force to widen the gingival sulcus, are conventionally used in dental therapeutics for widening the gingival sulcus. US patent No. 4,465,462 discloses a gingival retraction cord having a tapered diameter throughout its length and having a length sufficient to enable the cord to be wrapped several times about a tooth; US patent No. 4,522,593 discloses a gingival retraction cord having improved characteristics advantageously constructed by a knitting process; US patent No. 4,321,038 discloses a braided gingival cord; US patent No. 4,892,482 discloses a gingival tissue retraction cord made of strands impregnated with suitable therapeutic preparations. However, the pressure provided by these conventional designs of gingival cords cannot effectively prohibit haemorrhages or oozing from gingival tissue. Therefore, mechanochemical gingival retraction methods, which use gingival retraction cords in association with various astringent and/or hemostasis agents, are developed. For example, US patent No. 4,617,950 discloses an astringent gingival retraction cord that is coated with an astringent gel containing an astringent salt; US patent No. 5,635,162 discloses a hemostatic dental composition for controlling oral bleeding or providing gingival tissue fluid control; US patent No. 5,785,955 discloses a hemostatic composition for controlling oral bleeding or providing gingival tissue fluid control, which includes a hemostatic agent, a chemical agent for reducing the acidic activity of the hemostatic agent, and an aqueous base; US patent No. 6,568,398 discloses hemostatic therapeutic compositions applicable for gingival retraction cords; US patent No. 6,375,461 discloses a gingival retracting cord including propylhexedrine for providing hemostasis and retraction or displacement of gingival tissue; US patent No. 4,597,960 discloses an astringent hemostatic composition comprised of a micron-sized astringent hemostatic agent microencapsulated in a biocompatible and non-allergenic hemostatic polymer soluble in body fluids; US patent No. 4,260,597 discloses thermally reversible dental astringent gels.

In concept, a gingival retraction cord is easy to use. In actual practice, it requires a skillful technique to insert a gingival retraction cord into gingival sulcus. Devices and methods relative to gingival retraction cord operation improvement are numerous and requisite. For example, US patent No. 5,899,694 relates to the methods and apparatus for placing a loop of gingival retraction cord around the base of a tooth; US patent No. 5,480,303 discloses a gingival retraction cord tool for forming the retraction cord into a closed loop; US patent No. 4,854,867 discloses a dental tool for facilitating gingival retraction; US patent No. 6,575,749 discloses a gingival retractor including an elongate handle and a horseshoe-shaped guard at an operative end of the handle; US patent No. 5,540,588 discloses a dental retraction cord having a thermoplastic material such as polytetrafluoroethylene, i.e. PTFE or Teflon; US patent No. 4,232,688 relates to a gingival retraction cord dispenser and US patent No. 5,358,403 relates to a gingival retraction cord applicator.

Gingival retraction operation using retraction cord is skillful and time consuming. Basal anesthesia is necessary before operation. Normally, it takes about 7-10 minutes for the positioning of a gingival retraction cord around one single teeth. Six to ten days are needed for recovery after gingival retraction operation. According to reports, an average 0.1mm marginal recession is seen if a gingival retraction cord is stayed in gingival sulcus over 15 minutes. The problem becomes serious when gingival retraction is necessary for several teeth at a time. Therefore, cordless gingival retraction techniques are disclosed. For example, US patent No. 4,930,920 discloses a combined material applicator and gingival tissue retractor for making dental impressions. The combined material applicator and gingival tissue retractor facilitates dental impressions, however it is not suitable for dental impressions on regions going to bleed because impression materials are sensitive to water and astringent contains soluble salt.

Further, US patent No. 5,362,495 discloses an insert material for widening the gingival sulcus that takes the form of a biocompatible paste which is injectable for external use and preferably hydrophilic, whose viscosity is of between about 13000 and about 30000 Pascals.second. However, commercialized materials based on this invention contains aluminum chloride astringent more than 15wt% to provide the desired gingival retraction effect.

During a mechanochemical gingival retraction operation, chemical substance effectively causes tissue or blood vessels to contract and to further control oozing of gingival tissue fluid or blood. However, the application of chemical substance may cause trauma or side effects. For example, when astringent epinephrine (8%) is used, it causes increased blood pressure, accelerated heart rate, and periodontal tissue trauma. Researches show severe damage to gingival tissues upon application of aluminum chloride astringent agent (10wt%). Gingival retraction materials containing high concentration (over 15wt%) aluminum chloride astringent causes damage to gingival tissues, and are technically biased from the spirit of the design and techniques of the patent (US patent No. 5,362,495).

US 2003/194380 is directed to a periodontal regeneration composition comprising bone growth material and free collagen and optionally a thickener for increasing viscosity. The composition is to be filled into the periodontal pocket, i.e. a gingival recession with root exposure. The regeneration composition eventually fills out the cavity, meaning that it combines the root and alveolar bone, which is to be stimulated to be remodelled by the composition.

### SUMMARY OF THE INVENTION

The present invention therefore has an objective to provide a new gingival retraction material for solving the aforesaid drawbacks. Particularly, the present invention has an objective to provide a gingival retraction material that is capable of associating low concentration of astringent so as to prevent the periodontal tissue trauma.

To achieve the above-mentioned objectives of the invention, the present invention provides an injectable gingival retraction material (paste), which comprises an astringent and fibrillated fibers having high aspect ratio, i.e. length/diameter ratio, to increase the viscosity thereof up to 31x10³ to 71x10³ Pa·s (31x10⁶ to 71x10⁶ cP) so as to provide sufficient yield strength for gingival retraction. Since the needed viscosity of gingival retraction material for retracting gingival can be achieved under low solid-contained condition, the average dispersion distance of aluminum chloride ions of the astringent is shortened when the chemical astringent is added into the gingival retraction material of the present invention. Therefore, the gingival retraction material provided by the present invention can effectively prohibit haemorrhage or oozing from gingival tissue with low concentration aluminum chloride astringent such that the gingival retraction material provided by the present invention achieves the desired gingival retraction effect with less trauma of periodontal tissue.

The gingival retraction material of the present invention can widely be employed to the gingival retraction procedure prior to various dental treatments, for example, Class II or Class IV's cavity filling, ceramic adhesion, impression taking of crown and bridge, and Maryland Bridge adhesion. The gingival retraction material of the present invention is easy to use, saves much time in dental treatment, eliminates basal anesthesia, and prevents side effect of gingiva obsolescence due to improver application of force during gingival retraction.

### BRIEF DESCRIPTION OF THE DRAWINGS

FIG. 1 illustrates the configuration of fibrillated fibers according to the present invention.
FIG 2 illustrates the configuration of kaolin.
FIC. 3 is a release speed comparison chart of different gingival retraction materials of different aluminum chloride content.

### DETAILED DESCRIPTION OF THE INVENTION

The invention relates to a fibrillated fiber-contained gingival retraction material for injection application. This gingival retraction material contains fibrillated fibers, which have the viscosity improving property, astringent, and excipients, if desired, including taste-modifying agent, color agent, and filler, thereby increasing the material viscosity up to 31.0x10³ to 71.0x10³ Pa·s (31.0×10⁶ to 71.0×10⁶ cP), lowing the solid content of the material, and improving the transmission speed of astringent to facilitate dispersion of aluminum chloride over the surface of gingiva. After insertion into the patient's gingival sulcus, the retaining time of the gingival retraction material is about 0.5-2 minutes that is adjustable subject to thickness of the gingiva and health status of the patient. By means of material viscosity to provide sufficient yielding strength to widen the patient's gingival sulcus and by means of the feature of the gingival retraction material to lower the yield strength when wet, washing after insertion of the gingival retraction material in the patient gingival sulcus controls hemostasis and oozing of the gingival tissue, keeping the gingival sulcus dry for further impressions. The fibrillated fiber-contained gingival retraction material shows superior gingival retraction effect to conventional techniques. The invention provides faster astringent release speed, lowers the amount of aluminum chloride of astringent, and reduces damage of chemical substance to tissue.

The fibrillated fibers used in the present invention preferably have one or more shapes selected from the group consisting of ribbon, fibril, barb and a combination thereof. The length to diameter ratio of the fibrillated fibers is greater than 10. In addition, the fibrillated fibers can be made from a material selected from the group consisting of cellulose, poly para-pheneylene isophthalamine (PPT-A), and polyacrylonitrile (PAN). The content of the fibrillated fibers preferably ranges from 0.1 wt% to 15wt%, and more preferably from 2.0wt% to 10.0wt%.

The filler used in the present invention can be a sheet clad selected from the group consisting of talc, mica, kaolin and montmorillonite. Preferably, the filler is kaolin. The content of the kaolin preferably ranges from 30.0wt% to 65.0wt%, and more preferably from 30.0wt% to 50.0wt%.

The astringent, which is contained in the gingival retraction material of the present invention, can be one or more compounds selected from the group consisting of aluminum acetate, aluminum chloride, aluminum sulfate, ferrous sulfate, zinc chloride and a mixture thereof. Preferably, the astringent used in the present invention is an aluminum chloride astringent. The content of the aluminum chloride astringent ranges from 7.0wt% to 15.0wt%, preferably from 7.0wt% to 10.0wt%.

Now the features of the present invention and the method for making the gingival retraction material will be discussed in more detail in terms of examples. It is to be understood at the outset of the description that persons of skill in the appropriate arts may modify the invention described here while still achieving the favorable results of this invention. Accordingly, the following description is to be understood as being a broad, teaching disclosure directed to persons of skill in the appropriate arts, and not as limiting upon the present invention.

### <EXAMPLE 1>

Preparation of fibrillated fibers: cellulose fibers of 2 deniers fineness were used to make a bundle of 1,000,000 deniers of 1 meter long, then a high-speed chopping machine was used to chop the bundle of cellulose fibers into chopped fiber strands of 6 mm long, and then a disk grinder was used to process the chopped stands into fibrillated fibers having the shapes of ribbon, fibrils, barbs, or their combination. FIC. 1 shows the typical shape of the fibrillated fibers used in the present invention. The aspect ratio (L/D) of the fibrillated fibers, which is defined as the ratio of the length L to the diameter D of the fibrillated fibers, is greater than 10. Under CSF (Canadian Standard Freeness) test subject to TAPPI T-227, the CSF value of the fibrillated fibers was 102 ml.

Preparation of fibrillated fiber-contained gingival retraction material: Added 0.2g astringent, for example, aluminum chloride to 4g water to form a solution, and then added 2g fibrillated fibers to the solution, and then used a mixer to well mix the fibrillated fibers with the solution for about 10 minutes, ensuring unfastening of micro fibers of the fibrillated fibers, and then added 3.8g taste-modifying agent, for example, sodium carbonate and 10g kaolin as filler to the mixture thus obtained and well mixed the mixture in Algimax^{™} for 20 seconds to form the desired fibrillated fiber-contained gingival retraction material.

During viscosity test, took 4.0g prepared fibrillated fiber-contained gingival retraction material and filled it into the standard jacket-type sample container of a Brookfield HBDVII+ viscometer and compacted the material with the attached pusher to a predetermined height, and at the same time controlled the circulation water bath to maintain the temperature of the sample container at 20°C, and then used the T-F type spindle of the viscometer to measure the viscosity and recorded the average of the measured values. The rotational speed of the spindle was set at 0.7 rpm and 0.8 rpm.

### <EXAMPLES 2-10>

Fibrillated fiber-contained gingival retraction materials were prepared subject to the procedure and method of the above-mentioned Example 1, except that the weight percentages of the aluminum chloride, fibrillated fibers, sodium carbonate, kaolin, and water are respectively defined as the following Table 1. Table 1 also shows the measured result of the viscosities of Examples 1-10.

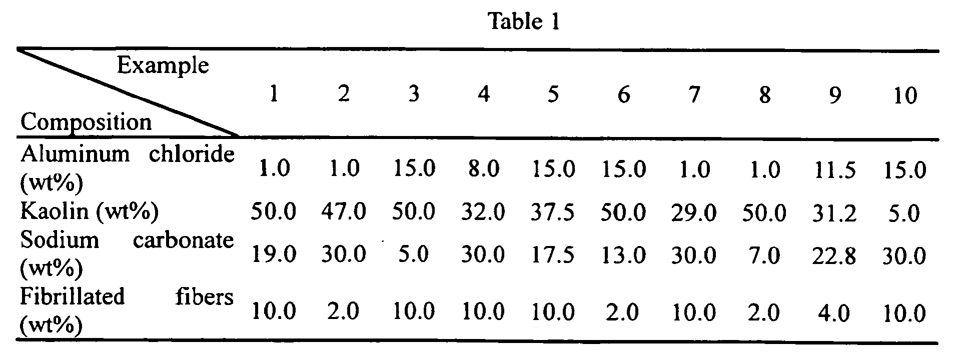

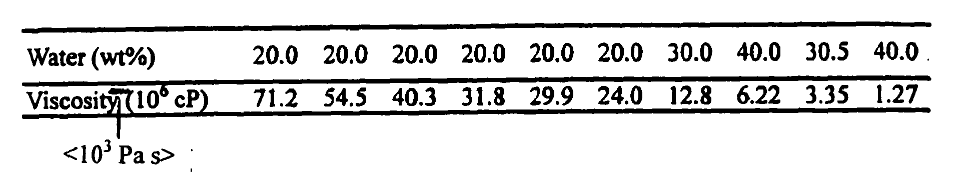

In early days, gingival retraction was achieved by a simple mechanical way with the use of a pure gingival retraction cord. Recently, US patent No. 5,362,495 teaches the use of an insert material for widening the gingival sulcus, which material takes the form of a biocompatible paste containing 3.6-6.8 wt% aluminum chloride astringent which is injectable for external use and preferably hydrophilic, whose viscosity is of between about 13.0x10³ and 30.0x10³ Pa·s (13.0×10⁶ and 30.0×10⁶ cP). The added chemical substance improves gingival retraction effect, however it may injure gingival tissues. Therefore, following examples were prepared at different viscosity without or with aluminum chloride astringent, and evaluated by animal test.

### <COMPARATIVE EXAMPLES 1 ADN 2>

Prepared fibrillated fiber-contained gingival retraction material comparative examples based on gingival retraction material viscosity between about 13.0x10³ and 30.0x10³ Pa·s (13.0×10⁶ and about 30.0x 10⁶ cP) as disclosed in US patent No. 5,362,495 and by means of the procedure and method of the aforesaid Example 1, except that the contents of aluminum chloride astringent, fibrillated fibers, sodium carbonate, kaolin, and water were determined subject to Table 2. Evaluation results of animal tests on gingival retraction effect and gingiva obsolescence were recorded and shown in Table 2.

The animal test procedure for evaluation of gingival retraction effect comprised the following steps.
1. Chose 5 sample Beagle dogs weighing from 9kgs through 15 kgs, and took care and examined oral health and status of gingival of chosen sample Beagle dogs regularly subject to related rules.
2. Drilled a reference positioning point between the mid point of the buccal region and the marginal gingiva before gingival retraction of the sample Beagle dogs, and then impressed polyvinyl siloxane on the arch of the tooth around the upper right, upper left, lower left, and lower right corners to make four molds, and then operated a 20 times reflective microscope Mitutoyo PJ-2500 Profile Projector and a meter (resolution 0.001mm) to measure and record the distance between the reference point and the marginal gingiva before gingival retraction.
3. Selected sample Beagle dogs randomly and marked selected sample Beagle dogs with a respective code number, and then defined the mouth of each selected sample Beagle dog into four quadrants subject to designed gingival retraction materials, and then applied different gingival retraction materials to selected four teeth (first molar, second molar, fourth premolar, and stomach teeth) in every quadrant (every sample Beagle dog had 16 sampling points) by means of using a hypodermic syringe to inject the respective gingival retraction material into the respective gingival sulcus at injection speed of 2mm/sec., and then washed gingival retraction material away from the premolar 2 minutes after injection, and then dried the washed area with air, and then impressed polyvinyl siloxane on the arch to make the desired gypsum molds, and then cut off every premolar gypsum mold at the mid point between the distal and the mesial along the major axis of the teeth, and then operated a 20 times reflective microscope and a meter to measure gingival sulcus width between the vertical surface of the teeth and the top end of the marginal gingival by means of contour profile, and then evaluated the gingival retraction effect of every gingival retraction material subject to the distance change before and after gingival retraction.
4. Polyvinyl siloxane was used to make molds corresponding to tooth arch region in every quadrant 2 weeks after gingival retraction, and then a 20 times reflective microscope and a meter were used to measure the vertical distance between the marginal gingival and the bottom side of the mark by means of contour profile, and then recorded distance changes between the measurements obtained before application of gingival retraction and the measurements obtained 2 weeks after application of gingival retraction, and then evaluated the effects of different gingival retraction materials at gingiva by means of statistics' paired t-test.
5. According to Alber (Alber Harry F. Impressions. A Text for Selection of Materials and Techniques. Santa Rose, Calif: Alto Books, 1990:21), gingival sulcus width should be greater than 0.5mm upon the use of rubber impression material. Therefore, the gingival retraction material that can widen the gingival sulcus more than 0.5mm will be determined to be effective. Paired t-test techniques were used to analyze the gingival sulcus widths and the vertical distance between the marginal gingiva and the bottom side of the mark, and statistically significant differences (P<0.05) before and after gingival retraction were defined to be effective in statistics.

### <EXAMPLES 11-12>

Fibrillated fiber-contained gingival retraction materials were prepared according to the procedure and method of Example 1, except that the weight percentages of the aluminum chloride, fibrillated fibers, sodium carbonate, kaolin, and water are respectively defined as the Table 2. Viscosities of the prepared gingival retraction materials were examined in the same manner as Example 1. Evaluation on retraction effect on each material was made subject to the animal test procedure of Comparative Example 1. Table 2 shows viscosities of different gingival retraction materials, their retraction effects and related animal test evaluation results.

The test results show gingival sulcus width to be greater than 0.5mm after the use of Examples 11 and 12, i.e., high-viscosity gingival retraction materials 31.0x10³ and 54.0x10³ Pa·s (31.0×10⁶ and 54.0×10⁶ cP) provide effective gingival retraction effect that was superior to Comparative Examples 1 and 2; there was no statistically significant difference on the height of marginal gingiva before and after gingival retraction, i.e., no gingiva obsolescence.

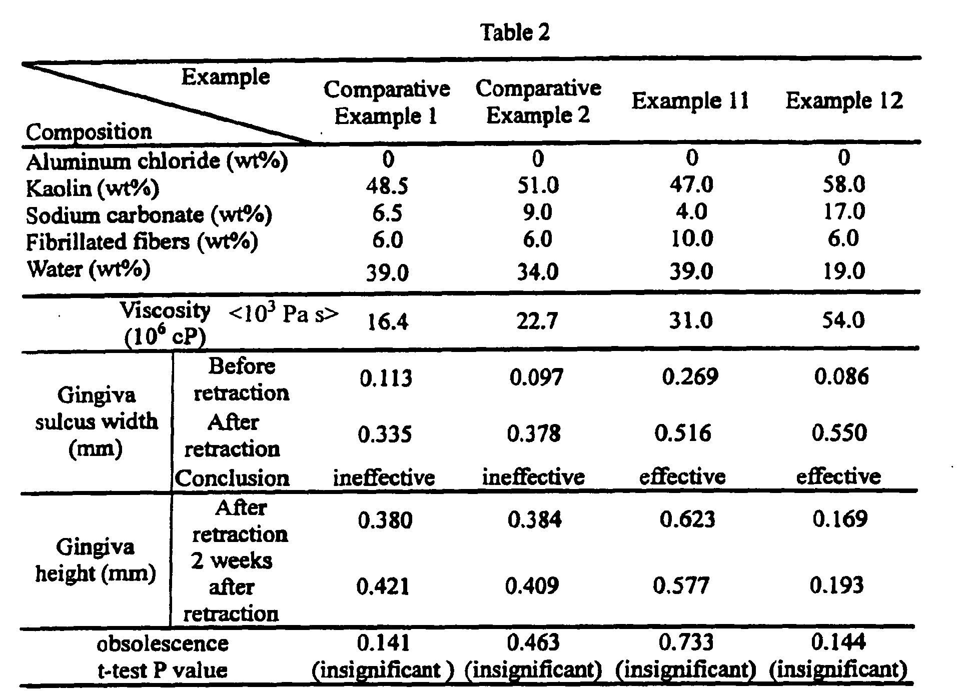

### <COMPARATIVE EXAMPLES 3-5>

Prepared fibrillated fiber-contained gingival retraction material comparative examples based on gingival retraction material viscosity between about 13.0x10³ to 30.0x10³ Pa·s (13×10⁶ to 30×10⁶ cP) as disclosed in US Pat. 5,362,495 and by means of the procedure and method of the aforesaid Example 1 with 3.6-6.8wt% aluminum chloride astringent and the weight percentage of fibrillated fibers, sodium carbonate taste-modifying agent, kaolin filler, and water determined subject to the following Table 3. Evaluation results of animal tests on gingival retraction effect and gingiva obsolescence were recorded and shown in the Table 3.

### <EXAMPLES 13>

Fibrillated fiber-contained gingival retraction materials were prepared subject to the procedure and method of Example 1, however the weight percentage of aluminum chloride astringent, fibrillated fibers, sodium carbonate taste-modifying agent, kaolin filler, and water were changed subject to the Table 3. Gingival material viscosity tests were performed in the same manner as the aforesaid Example 1. Evaluation on effect of gingival retract materials was made subject to the animal test procedure of the aforesaid Comparative Example 1. Viscosity values of different gingival retraction materials are shown in Table 3.

Paired t-test method was employed to analyze gingival sulcus width and height of marginal gingiva before and after gingival retraction. Results showed 15wt% aluminum chloride astringent should be added to Example 13 to achieve the desired effect of gingival sulcus width, i.e. more than 0.5 mm after gingival retraction, when viscosity at 23.0x10³ Pa·s (23.0×10⁶ cP). That was superior to the techniques of Comparative Examples 3-5 (0, 5, 10wt% aluminum chloride astringent). There were no statistically significant differences on height of marginal gingiva before and after gingival retraction, i.e., no gingiva obsolescence.

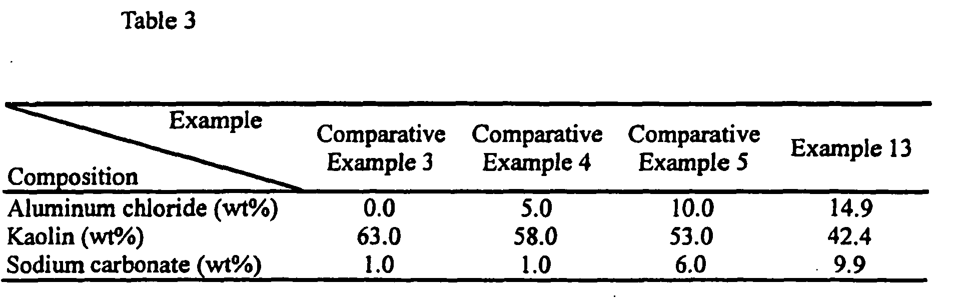

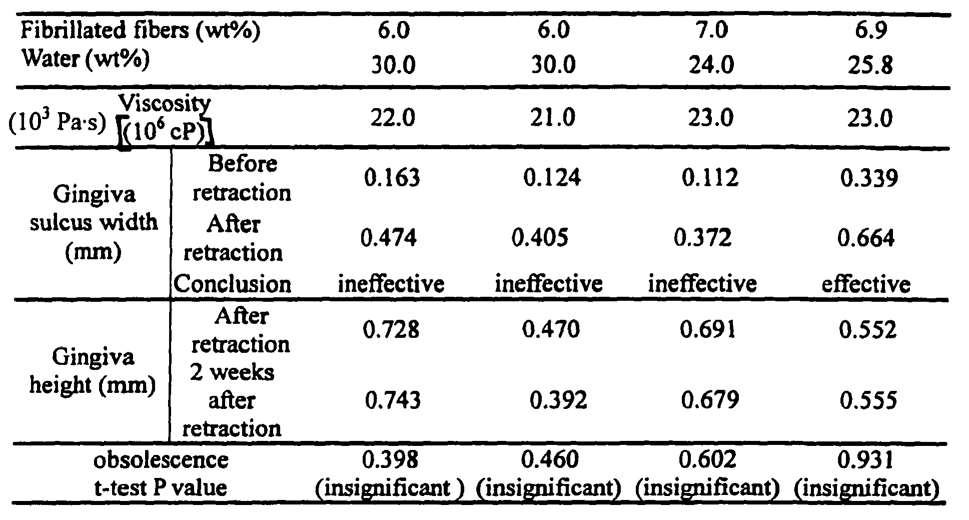

### <COMPARATIVE EXAMPLE 6>

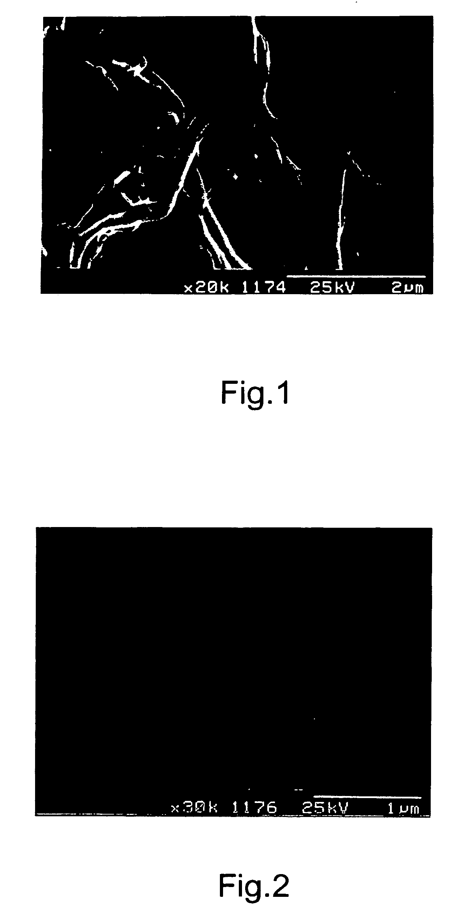

Prepared Comparative Example 6 containing 15wt% aluminum chloride astringent based on Expa-syl^{®} from Krr Corporation with flat sheet kaolin, as shown in FIG. 2, added as viscosity improver to provide thrust force 0.1N/mm², and then applied the material to gingival by means of mechanochemical method. Evaluated the release speed of the aluminum chloride of gingival retraction material by means of examining the relation between iron dispersion speed and conductivity. The operation procedure was as follows:

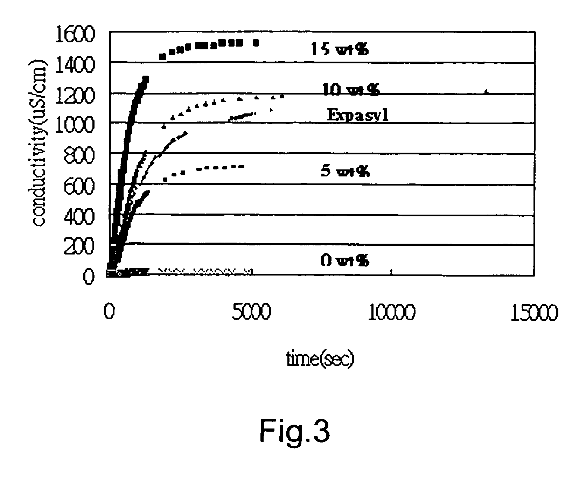

In order to control same contact area of the gingival retraction material in the test during release of ions, 1.0g gingival retraction material sampling amount was filled in a 1ml syringe Tuberculinl^{®}, and then the sample was squeezed into a cord-like material, which was then packed in paper filter to maintain material intact during test, and then the package of paper filter was put in a beaker containing 200ml ultra pure water and stirred with a magnet, and then insert the probe of a portable conductivity meter Suntex SC-120 into the solution, and then measured and recorded conductivity-time variation. The test results were shown in FIG. 3.

### <EXAMPLES 14-17>

Fibrillated fiber-contained gingival retraction materials were prepared based on Expa-syl^{®} from Krr Corporation and subject to the procedure and method used for the aforesaid Example 1 with fibrillated fibers used to partially substitute for kaolin and changed aluminum chloride to concentrations 0%, 5%, 10% and 15% respectively, as shown in Table 4. Evaluation on release speed of the aluminum chloride of gingival retraction materials was made subject to the operation procedure of the aforesaid Comparative Example 6. Conductivity-Time variation results were shown in FIG. 3.

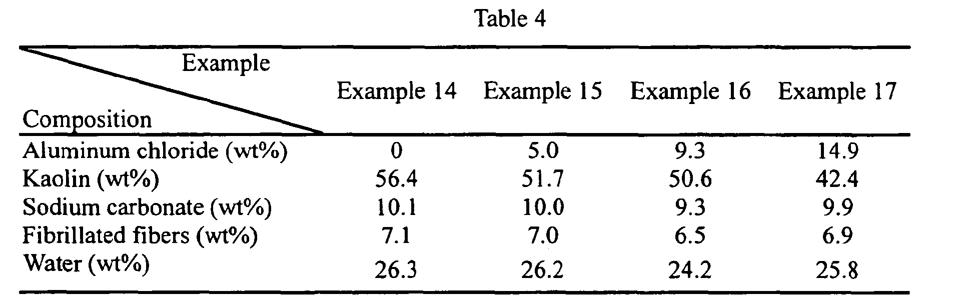

According to variation of conductivity relative time of Example 14 (0wt% aluminum chloride astringent), the existence of other compositions in the gingival retraction material showed no effect on measuring of conductivity. When aluminum chloride content rose, increasing speed of conductivity and equilibrium conductivity were relatively increased. FIG. 3 shows that the conductivity raising speed and equilibrium conductivity curve of the Comparative Example 6 (Expa-syl®) containing 15wt% aluminum chloride is located in the region between the curves of Examples 15 and 16 (5-10wt% aluminum chloride astringent). It shows that the use of fibrillated fiber as viscosity improver to substitute for kaolin effectively increase release speed of aluminum chloride astringent, thereby achieving sample release amount as Comparative Example 6 with a relatively lower astringent concentration to prevent injury to gingiva tissues.

## Claims

1. A gingival retraction material, which is a paste material injectable into the gingival sulcus of a patient for widening the patient's gingival sulcus, comprising an aluminum chloride astringent in the range from 7.0wt% to 15.0wt% and fibrillated fibers having a length/diameter ratio greater than 10, wherein said gingival retraction material has a viscosity at 20°C ranging from 31.0x10³ Pa·s (31.0x10⁶ cP) to 71.0x10³ Pa·s (71.0x10⁶ cP).

2. The gingival retraction material as claimed in claim 1, further comprising water, taste-modifying agent, color agent, and filler.

3. The gingival retraction material as claimed in claim 2, wherein said fibrillated fibers have one or more shapes selected from the group consisting of ribbon, fibril, barb and a combination thereof.

4. The gingival retraction material as claimed in claim 2, wherein said fibrillated fibers are made from a material selected from the group consisting of cellulose, poly para-pheneylene isophthalamine (PPT-A), and polyacrylonitrile (PAN).

5. The gingival retraction material as claimed in claim 2, wherein a content of said fibrillated fibers ranges from 0.1 wt% to 15wt%.

6. The gingival retraction material as claimed in claim 5, wherein the content of said fibrillated fibers ranges from 2.0wt% to 10.0wt%.

7. The gingival retraction material as claimed in claim 2, wherein said filler is a sheet clad selected from the group consisting of talc, mica, kaolin and montmorillonite.

8. The gingival retraction material as claimed in claim 2, wherein said filler is kaolin, and a content of said kaolin ranges from 30.0wt% to 65.0wt%.

9. The gingival retraction material as claimed in claim 8, wherein the content of said kaolin ranges from 30.0wt% to 50.0wt%.

10. The gingival retraction material as claimed in claim 1, wherein the content of said aluminum chloride astringent ranges from 7.0wt% to 10.0wt%.

## Patentansprüche

1. Zahnfleisch-Retraktionsmaterial, das ein in den Zahnfleisch-Sulcus eines Patienten zum Weiten des Zahnfleisch-Sulcus des Patienten injizierbares Pastenmaterial ist, welches umfasst, ein Aluminiumchlorid-Astringens im Bereich von 7 Gew.-% bis 15 Gew.-% und ein faseriges Fasermaterial mit einem Längen/Durchmesser-Verhältnis von über 10, worin das Zahnfleisch-Retraktionsmaterial eine Viskosität bei 20 °C im Bereich von 31,0 x 10³ Pa·s (31,0x 10⁶ cP) bis 71 x 10³ Pa·s (71,0 x 10⁶ cP) aufweist.

2. Zahnfleisch-Retraktionsmaterial nach Anspruch 1, welches weiter Wasser, Geschmacks-verändernde Mittel, Färbemittel und Füllmaterial enthält.

3. Zahnfleisch-Retraktionsmaterial nach Anspruch 2, worin das faserigen Fasermaterial ein oder mehrere Formen aufweist, ausgewählt aus der Gruppe bestehend aus Bändern, Fibrillen, Widerhaken und eine Kombination davon.

4. Zahnfleisch-Retraktionsmaterial nach Anspruch 2, worin das Fibrillen-Fasermaterial aus einem Material hergestellt ist, ausgewählt aus der Gruppe bestehend aus Cellulose, Polyparaphenylenisophthalamin (PPT-A) und Polyacrylnitril (PAN).

5. Zahnfleisch-Retraktionsmaterial nach Anspruch 2, worin der Gehalt an Fibrillen-Fasermaterial im Bereich von 0,1 Gew.-% bis 15 Gew.-% liegt.

6. Zahnfleisch-Retraktionsmaterial nach Anspruch 5, worin der Gehalt an Fibrillen-Fasermaterial im Bereich von 2,0 Gew.-% bis 10,0 Gew.-% liegt.

7. Zahnfleisch-Retraktionsmaterial nach Anspruch 2 ,worin das Füllmaterial ein Lagen-Überzug ist ausgewählt aus der Gruppe bestehend aus Talkum, Mika, Kaolin und Montmorillonit.

8. Zahnfleisch-Retraktionsmaterial nach Anspruch 2, worin der Gehalt an Kaolin im Bereich von 30,0 Gew.-% bis 65,0 Gew.-% liegt.

9. Zahnfleisch-Retraktionsmaterial nach Anspruch 8, worin der Gehalt des Kaolins im Bereich von 30,0 Gew.-% bis 50,0 Gew.-% liegt.

10. Zahnfleisch-Retraktionsmaterial nach Anspruch 2, worin der Gehalt an Aluminiumchlorid-Astringens im Bereich von 7,0 Gew.-% bis 10,0 Gew.-% liegt.

## Revendications

1. Matériau de rétraction gingivale, constitué d'un matériau pâteux injectable dans le sillon gingival d'un patient pour élargir le sillon gingival du patient, comportant un astringent de chlorure d'aluminium compris entre 7,0 pourcent en poids et 15,0 pourcent en poids et des fibres fibrillées d'un rapport longueur/diamètre supérieur à 10, **caractérisé en ce que** ledit matériau de rétraction gingivale présente une viscosité à 20°C allant de 31,0 x 10³ Pas (31,0 x 10⁶cP) à 71,0 x 10³ Pas (71,0 x 10⁶ cP).

2. Matériau de rétraction gingivale selon la revendication 1, comprenant en outre de l'eau, un agent modificateur de goût, un agent colorant et une matière de remplissage.

3. Matériau de rétraction gingivale selon la revendication 2, **caractérisé en ce que** lesdites fibres fibrillées possèdent une ou plusieurs formes sélectionnées parmi le groupe composé de ruban, fibrille, barbe et une combinaison de ceux-ci.

4. Matériau de rétraction gingivale selon la revendication 2, **caractérisé en ce que** lesdites fibres fibrillées sont réalisées en un matériau sélectionné parmi le groupe composé de cellulose, poly-paraphénylène- isophthalamine (PPT-A), et polyacrylonitrile (PAN).

5. Matériau de rétraction gingivale selon la revendication 2, dans lequel la teneur desdites fibres fibrillées est comprise entre 0,1 pourcent en poids et 15 pourcent en poids.

6. Matériau de rétraction gingivale selon la revendication 5, dans lequel la teneur desdites fibres fibrillées est comprise entre 2,0 pourcent en poids et 10,0 pourcent en poids.

7. Matériau de rétraction gingivale selon la revendication 2, dans lequel ladite matière de remplissage est un astringent de chlorure d'aluminium revêtu en feuille, constituant ainsi une quantité de libération comme dans l'exemple comparatif 6 avec une concentration d'astringent relativement faible pour protéger les tissus gingivaux sélectionné parmi le groupe composé de talc, mica, kaolin et montmorillonite.

8. Matériau de rétraction gingivale selon la revendication 2, dans lequel ladite matière de remplissage est le kaolin, et la teneur dudit kaolin est comprise entre 30,0 pourcent en poids et 65,0 pourcent en poids.

9. Matériau de rétraction gingivale selon la revendication 8, dans lequel la teneur dudit kaolin est comprise entre 30,0 pourcent en poids et 65,0 pourcent en poids.

10. Matériau de rétraction gingivale selon la revendication 1, dans lequel la teneur dudit astringent de chlorure d'aluminium est comprise entre 7,0 pourcent en poids et 10,0 pourcent en poids
